(19) Europäisches Patentamt

European Patent Office

Office européen des brevets

(11) **EP 1 150 251 B1**

(12) **EUROPÄISCHE PATENTSCHRIFT**

(45) Veröffentlichungstag und Bekanntmachung des Hinweises auf die Patenterteilung:
**08.03.2006 Patentblatt 2006/10**

(51) Int Cl.:
***G06T 11/00*** *(2006.01)*

(21) Anmeldenummer: **01000125.3**

(22) Anmeldetag: **26.04.2001**

(54) **Computertomographie-Verfahren**

Computer tomography method

Méthode de tomographie assistée par ordinateur

(84) Benannte Vertragsstaaten:
**DE FR GB NL**

(30) Priorität: **29.04.2000 DE 10021219**

(43) Veröffentlichungstag der Anmeldung:
**31.10.2001 Patentblatt 2001/44**

(73) Patentinhaber:
  • **Philips Intellectual Property & Standards GmbH**
    **20099 Hamburg (DE)**
    Benannte Vertragsstaaten:
    **DE**
  • **Koninklijke Philips Electronics N.V.**
    **5621 BA Eindhoven (NL)**
    Benannte Vertragsstaaten:
    **FR GB NL**

(72) Erfinder:
  • **Grass, Michael**
    **52064, Aachen (DE)**

  • **Koehler, Thomas**
    **52061 Aachen (DE)**
  • **Proksa, Roland**
    **52061 Aachen (DE)**

(74) Vertreter: **Volmer, Georg et al**
    **Philips Intellectual Property & Standards GmbH,**
    **Postfach 50 04 42**
    **52088 Aachen (DE)**

(56) Entgegenhaltungen:
    **DE-A- 19 843 812     US-A- 5 430 783**
    **US-A- 5 444 792**

  • **GRASS M ET AL: "3D CONE-BEAM CT RECONSTRUCTION FOR CIRCULAR TRAJECTORIES" PHYSICS IN MEDICINE AND BIOLOGY, TAYLOR AND FRANCIS LTD. LONDON, GB, Bd. 45, Nr. 2, Februar 2000 (2000-02), Seiten 329-347, XP000993403 ISSN: 0031-9155**

**Beschreibung**

Die Erfindung betrifft ein Computertomographie-Verfahren mit den Schritten

- Erzeugen eines kegelförmigen, einen Untersuchungsbereich bzw. ein darin befindliches Objekt durchsetzenden Strahlenbündels mit einer Strahlenquelle,
- Erzeugung von Relativbewegungen zischen der Strahlenquelle einerseits und dem Untersuchungsbereich bzw. dem Objekt andererseits, die eine Rotation um eine Rotationsachse entlang einer ersten, geschlossenen Trajektorie und entlang wenigstens einer zweiten mit der ersten identischen, jedoch in Richtung der Rotationachse versetzten Trajektorie umfassen,
- Akquisition von Meßdaten, die von der Intensität in dem Strahlenbündel jenseits des Untersuchungsbereiches abhängen, mit einer Detektoreinheit während der Relativbewegungen,
- Rekonstruktion der Abrorptionsverteilung im Untersuchungsbereich.

Außerdem bezieht sich die Erfindung auf einen Computertomographen, sowie ein Computerprogramm zur Steuerung eines solchen Computertomographen.

Die Verwendung eines kegelförmigen - anstelle eines fächerförmigen - Strahlenbündels hat den Vorteil, dass ein größerer Teil des Untersuchungsbereiches (gemessen in Richtung der Rotationsachse) bzw. des darin befindlichen Objektes mit einem einzigen Umlauf entlang der geschlossenen Trajektorie erfaßt und dargestellt werden kann. Soll ein noch größerer Teil des Untersuchungsbereichs abgebildet werden, muß nach einem solchen Umlauf mindestens einmal eine Relativverschiebung in Richtung der Rotationsachse erfolgen und der Untersuchungsbereich von einer zweiten Trajektorie aus durchstrahlt und abgebildet werden. Die Verschiebung muß dabei so groß gewählt werden, dass eine lückenlose Abbildung erfolgen kann.

Bei einem aus der deutschen Patentanmeldung 19 843 812.5 (PHD 98-111) bekannten Verfahren kann die Verschiebung so groß sein, dass die Voxel in einem Zwischenbereich weder von der einen Trajektorie aus noch von der anderen Trajektorie aus ständig von Röntgenstrahlung getroffen werden. Die Rekonstruktion der Absorptionsverteilung in diesem Zwischenbereich erfolgt dabei dadurch, dass man für jedes Voxel nur solche Meßdaten heranzieht, die einem Bestrahlungswinkelbereich von nur 180° - bezogen auf das Voxel - zugeordnet snd. Ein Nachteil dieses Verfahrens besteht darin, dass die Absorptionsverteilung in dem Zwischenbereich - im Vergleich zur Absorptionsverteilung in den beim Umlauf der Strahlenquelle entlang den Trajektorien ständig von Strahlung getroffenen Bereichen - nur mit einem ungünstigeren Signal-/Rauschverhältnis und zusätzlichen Artefakten rekonstruiert werden kann.

Dieser Nachteil ließe sich zwar dadurch vermeiden, dass man die Verschiebung zwischen den beiden Trajektorien so gering wählt, dass die Bereiche des Objektes, die von einer der Trajektorien aus ständig von Strahlung getroffen werden, unmittelbar aneinander angrenzen. Dies führt zu einer erhöhten Strahlungsbelastung, falls das Verfahren für eine medizinische Untersuchung angewandt wird, zumindest aber zu einer erhöhten thermischen Belastung der Strahlenquelle. Außerdem verlängert sich die Akquisitionszeit, die erforderlich ist, um einen in Richtung der Rotationsachse vorgegebenen Teil des Untersuchungsbereichs abzutasten.

Aufgabe der vorliegenden Erfindung ist es, ein Verfahren der eingangs genannten Art so auszugestalten, dass sch einerseits eine gute Bildqualität, andererseits aber auch eine geringe Dosisbelastung bzw. kurze Akquisitionszeiten zur Erfassung eines bestimmten Teils des Untersuchungsbereichs ergeben.

Diese Aufgabe wird erfindungsgemäß gelöst durch eine solche Wahl des Abstandes der Trajektorien voneinander, dass

- einerseits in einem Zwischenbereich zwischen den Trajektorien Voxel vorhanden sind, die bei beiden Relativbewegungen zeitweise nicht von Strahlung getroffen werden, und
- andererseits die Strahlenquelle beide Trajektorien stets derart auf die Detektoreinheit projiziert, dass die Projektionen den seitlichen Rand des für die Akquisition von
- Meßdaten benutzten Bereiches der Detektoreinheit schneiden,

und durch eine Rekonstruktion der Absorptionsverteilung im Zwischenbereich, bei der

Meßdaten herangezogen werden, die während der beiden Relativbewegungen akquiriert wurden.

Bei der Erfindung existiert also neben den Bereichen, deren Absorption von einer der Trajektorien aus vollständig gemessen werden kann, ein Zwischenbereich, in dem dies nicht möglich ist, weil die Voxel in diesem Bereich von der Strahlenquelle nicht während des gesamten Umlaufs auf der Trajektorie bestrahlt werden. Die Erfindung beruht auf der Erkenntnis, dass die Winkelbereiche, über die ein Voxel im Zwischenbereich von den beiden Trajektorien aus bestrahlt wird, bei der angegebenen Wahl des Abstands der Trajektorien zusammen mindestens 360° betragen, so dass es möglich ist, die Absorptionsverteilung auch in dem Zwischenbereich mit einer vergleichbaren Bildqualität zu rekonstruieren wie in den Bereichen, deren Absorptionsverteilung aus den auf einer einzigen Trajektorie akquirierten Meßdaten rekonstruiert werden - vorausgesetzt, dass man Meßdaten von beiden Trajektorien zur Rekonstruktion heranzieht.

Vorzugsweise wird der Abstand zwischen den benachbarten Trajektorien gemäß Anspruch 2 gewählt. Diese Wahl des Abstandes ist optimal. Bei einem geringeren Abstand wird die Strahlenbelastung vergrößert bzw. die Meßzeit verlängert und bei einem größeren Abstand (die Projektion der einen Trajektorie auf die Detektoreinheit schneidet dann den seitlichen Rand des für die Akquisition von Meßdaten benutzten Bereichs der Detektoreinheit nicht mehr) ist es nicht möglich, die Absorptionsverteilung in dem Zwischenbereich ohne Verlust an Bildqualität zu rekonstruieren.

Anspruch 3 beschreibt ein bevorzugtes Rekonstruktionsverfahren mit einem im Vergleich zu anderen Methoden geringen Rechenaufwand, das zu einer sehr guten Bildqualität führt. Für die Rekonstruktion der Absorption im Zwischenbereich werden dabei gefilterte Daten von Gruppen verwendet, die von unterschiedlichen Trajektorien aus akquiriert wurden. Dieses Rekonstruktionsverfahren ist an sich aus der deutschen Patentanmeldung..... (PHD 98-123) bzw. aus dem Artikel von Graß, Köhler, Proksa "3D cone-beam CT reconstruction for circular trajectories" in Phys. Med Biol 45 (2000) 329-347, bekannt. Die in Anspruch 4 angegebene Ausgestaltung dieses Verfahrens ermöglicht eine besonders einfache Rekonstruktion.

Anspruch 5 beschreibt eine Computertomographen zur Durchführung des erfindungsgemäßen Verfahrens. Die bevorzugte Ausgestaltung nach Anspruch 6 führt in Verbindung mit dem Rekonstuktionsverfahren nach Anspruch 3 bei dem in Anspruch 2 definierten Abstand dazu, dass der Rand des Strahlenbündels und die Projektion der einen Trajektorie auf der Detektoreinheit zusammenfallen. In diesem Fall erhält jedes Voxel zwischen den beiden Trajektorien gerade so viel Strahlung, wie es für die angestrebte Bildqualität erforderlich ist.

Anspruch 7 definiert ein Computerprogramm zur Steuerung eines Computertomographer nach Anspruch 5.

Die Erfindung wird im folgenden anhand der Zeichnungen näher erläutert. Es zeigen:

Fig. 1    einen Computertomographen mit dem das erfindungsgemäße Verfahren ausführbar ist,

Fig 2    ein Ablaufdiagramm des erfindungsgemäßen Verfahrens,

Fig. 3    die Lage der Trajektorien, relativ zueinander und zum Untersuchungsbereich,

Fig 4    die Projektion der Trajektorien auf die Detektoreinheit,

Fig 5    ein in einer Strahlenquelleposition erzeugtes kegelfömiges Strahlenbündel,

Fig 6    die durch das Rebinning gebildeten Strahlenfächer in parallelen Ebenen,

Fig 7    einen Querschnitt durch diese Strahlenfächer und

Fig. 8    die Teile der Trajektorien, von denen aus ein Voxel durchstrahlt wird im bezug auf dieses Voxel.

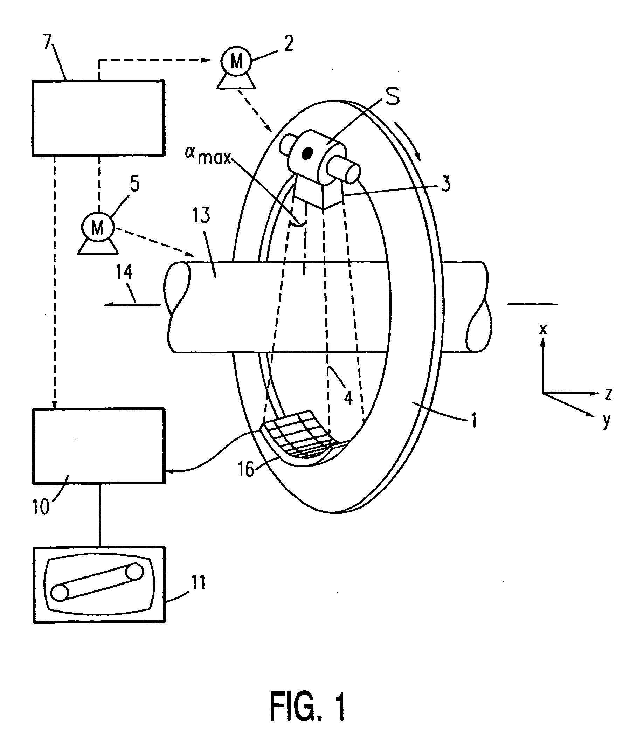

Der in Fig 1 dargestellte Computertomograph umfaßt eine Gantry 1, die um eine parallel zur z-Richtung des in Fig. 1 dargestellten Koordinatensystems verlaufende Rotationsachse 14 rotieren kann. Dazu wird die Gantry von einem Motor 2 mit einer vorzugsweise konstanten, aber einstellbaren Winkelgeschwindigkeit angetrieben. An der Gantry ist eine Strahlenquelle S befestigt, beispielsweise ein Röntgenstrahler. Dieser ist mit einer Kollimatoranordnung 3 versehen, die aus der von der Strahlenquelle S erzeugten Strahlung ein kegelförmiges Strahlenbündel 4 ausblendet, d.h. ein Strahlenbündel, das sowohl in z-Richtung als auch in einer dazu senkrechten Richtung (d.h. in einer zur Rotationsachse senkrechten Ebene) eine von Null verschiedene, endliche Ausdehnung hat.

Das Strahlenbündel 4 durchdringt einen Untersuchungsbereich 13, in dem sich ein Objekt, z.B. ein Patient auf einem Patientenlagerungstisch (beides nicht näher dargestellt) befinden kann. Der Untersuchungsbereich 13 hat die Form eines Zylinders. Nach dem Durchsetzen des Untersuchungsbereichs 13, trifft das Röntgenstrahlenbündel 4 auf eine an der Gantry 1 befestigte zweidimensionale Detektoreinheit 16, die eine Anzahl von Detektorzeilen mit jeweils einer Vielzahl von Detektorelementen umfaßt. Die Detektorzeilen befinden sich in zur Rotationsachse senkrechten Ebenen, vorzugsweise auf einem Kreisbogen um die Strahlenquelle S; sie können aber auch anders geformt sein, z.B. einen Kreisbogen um die Rotationsachse 14 beschreiben oder geradlinig sein. Jedes von dem Strahlenbündel 4 getroffene Detektorelement liefert in jeder Position der Strahlenquelle einen Meßwert für einen Strahl aus dem Strahlenbündel 4.

Der mit $\alpha_{max}$ bezeichnete Öffnungswinkel des Strahlenbündels 4 (als Öffnungswinkel ist der Winkel definiert, den ein Strahl, der in einer zur Rotationsachse 14 senkrechten Ebene am Rande des Strahlenbündels 4 liegt, mit einer durch die Strahlenquelle S und die Rotationsachse 14 definierten Ebene einschließt) bestimmt dabei den Durchmesser des Objektzylinders, innerhalb dessen sich das zu untersuchende Objekt bei der Akquisition der Meßwerte befindet. Der Untersuchungsbereich 13 - bzw. das Objekt oder der Patientenlagerungstisch - kann mittels eines Motors 5 parallel zur Rotationsachse 14 bzw zur z-Achse verschoben werden. Dazu äquivalent könnte aber auch die Gantry in diese Richtung verschoben werden.

Wenn die Motoren 5 und 2 gleichzeitig laufen, beschreiben die Strahlenquelle S und die Detektoreinheit 16 eine helixförmige Trajektorie relativ zum Untersuchungsbereich 13. Wenn hingegen der Motor 5 für den Vorschub in z- Richtung stillsteht und der Motor 2 die Gantry rotieren läßt, ergibt sich eine kreisförmige Trajektorie für die Strahlenquelle S und die Detektoreinheit 16 relativ zum Untetsuchungsbereich 13. Im folgenden soll wegen der Einfachheit der Erzeugung einer solchen kreisförmigen Trajektorie nur diese betrachtet werden, obwohl auch andere geschlossene Trajektorien, beispielsweise elliptische oder solche, die in bezug auf die Rotationsachse geneigt sind, möglich sind.

Die von der Detektoreinheit 16 akquirierten Meßdaten werden einem Bildverarbeitungsrechner 10 zugeführt, der daraus die Absorptionsverteilung in einem Teil des Untersuchungsbereichs 13 rekonstruiert und z.B. auf einem Monitor 11 wiedergibt. Die beiden Motoren 2 und 5, der Bildverarbeitungsrechner 10, die Strahlenquelle S und der Transfer der Meßdaten von der Detektoreinheit 16 zum Bildverarbeitungsrechner 10 werden von einer Kontrolleinheit 7 gesteuert.

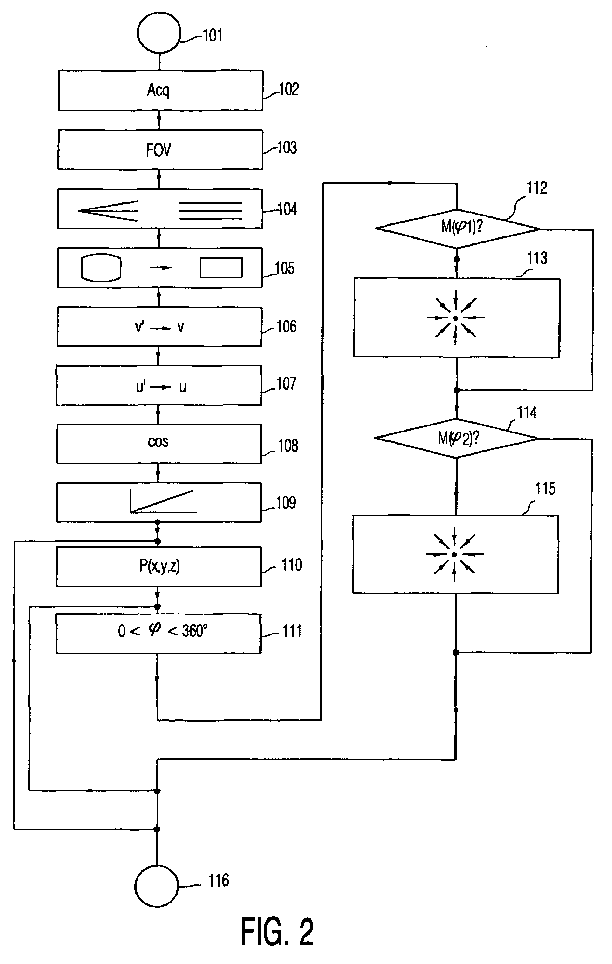

Fig. 2 zeigt den Ablauf eines Meß- und Rekonstruktionsverfahrens, das mit dem Computertomographen nach Fig. 1 durchgeführt werden kann.

Nach der Initialisierung im Block 101 rotiert die Gantry mit einer konstanten Winkelgeschwindigkeit. Im Schritt 102 wird der Durchmesser des darzustellenden Bereichs FOV (field of view) festgelegt. Der Durchmesser kann dem durch $\alpha_{max}$ definierten Maximaldurchmesser des Untersuchungsbereichs 13 entsprechen, er kann jedoch auch kleiner sein.

Im Schritt 103 wird die Strahlung der Strahlenquelle S eingeschaltet, und die auf dieser ersten Trajektorie von den Detektorelementen der Detektoreinheit 16 akquirieren Meßwerte werden in einem Speicher des Bildverarbeitungsrechners 10 gespeichert. Danach werden das Objekt bzw. der Untersuchungsbereich 13 einerseits und die Gantry 1 mit der Strahlenquelle S und der Detektoreinheit 16 relativ zueinander um eine Strecke d verschoben (wobei die Röntgenstrahlung ausgeschaltet ist). Danach rotiert die Gantry erneut mit konstanter Winkelgeschwindigkeit (entlang einer zweiten Trajektorie im bezug auf den Untersuchungsbereich). Die Röntgenstrahlung wird wieder eingeschaltet und die dabei von den Detektorelementen der Detektoreinheit akquirieren Meßwerte werden wiederum in dem Speicher des Bildverarbeitungsrechners 10 gespeichert.

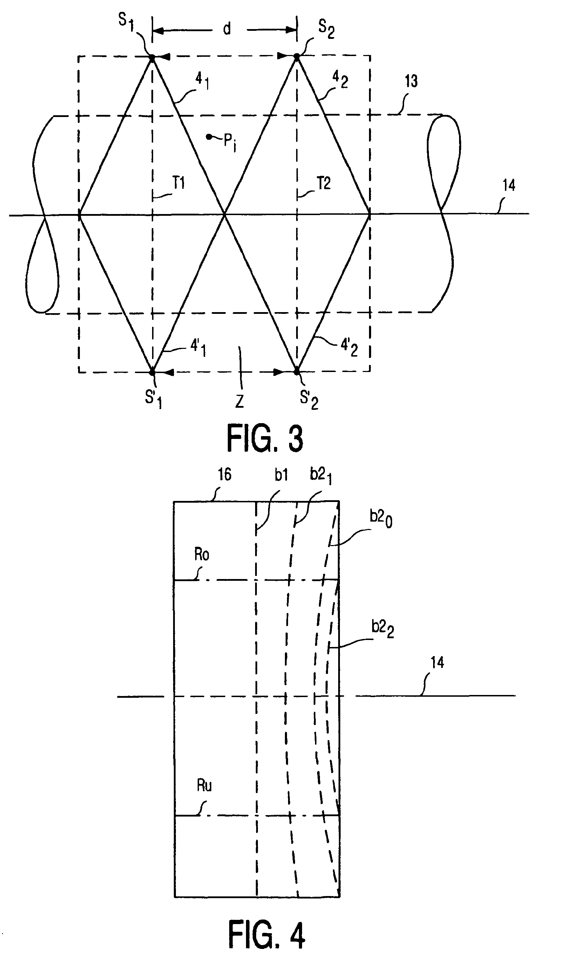

Fig 3 zeigt die Lage der kreisförmigen Trajektorien T1 und T2, auf denen sich die Strahlenquelle S relativ zum Untersuchungsbereich 13 bewegt. Dabei ist der Einfachheit halber angenommen worden, dass die Gantry bzw. die Trajektorie verschoben worden ist und das Objekt stillgestanden hat, doch ist dies irrelevant; wesentlich ist allein die Relativverschiebung zwischen Untersuchungsbereich 13 einerseits und Gantry andererseits. Die kreisförmigen Trajektorien T1 und T2 erscheinen als (gestrichelte) Geraden, weil die Rotationsachse 14 in der Darstellung der Fig. 3 in der Zeichenebene liegt. Außerdem ist die Strahlenquelle (als Punkt angedeutet) auf jeder Trajektorie in der höchsten Position ($S_1$ und $S_2$) und in der niedrigsten Position ($S'_1$ und $S'_2$) eingezeichnet. Für jede dieser Positionen ist mit ausgezogenen Linien das zugehörige Strahlenbündel ($4_1$ und $4_2$ bzw. $4'_1$ und $4'_2$) angedeutet. Die Trajektorien haben voneinander den Abstand d

Man erkennt in Fig 3 zwei durch die Strahlenbündel $4_1$ und $4_2$ bzw. $4'_1$ und $4'_2$ definierte diskusförmige Bereiche. In diesen Bereichen kann die Absorptionsverteilung vollständig mit Hilfe der Meßdaten rekonstruiert werden, die auf einer der beiden Trajektorien akquiriert wurden. Dazwischen liegt ein Bereich Z. Die Voxel in diesem Bereich werden bei den Umläufen der Strahlenquelle entlang der Trajektorien zeitweilig nicht von Strahlung getroffen. So wird beispielsweise das Voxel $P_i$ von den Strahlenquellenpositionen Position $S_1$ und $S_2$ nicht von Strahlung getroffen.

Wie der Abstand d gewählt werden soll, damit die Absorptionsverteilung auch im Bereich Z rekonstruiert werden kann, wird im folgenden anhand der Fig 4 erläutert. Fig 4 stellt die Projektion beider Trajektorien auf die Detektoreinheit 16 dar. Diese Darstellung beruht auf der Annahme, dass die immateriellen Trajektorien auf die Detektoreinheit projiziert werden können (in einer geometrischen Begriffswelt ist eine solche Projektion aber ohne weiteres vorstellbar) und dass die Detektoreinheit 16 sich auf einer der Trajektorien befindet, zB. auf der linken Trajektorie T1.

Die Projektion der Trajektorie T1 ist mit b1 bezeichnet. Sie ist - wie in Fig. 4 dargestellt - identisch mit der Mittellinie der Detektoreinheit, wenn davon ausgegangen wird, dass sich die Detektoreinheit 16 symmetrisch zu der Trajektorie erstreckt. Die Trajektorie T2 wird (bei einer Verschiebung nach rechts) grundsätzlich rechts von der Trajektorie T1 auf die Detektoreinheit 16 projiziert, wobei ihr Abstand von der Projektion b1 um so größer ist, je größer der Abstand d der Trajektorie T2 ist. Wenn die Detektoreinheit 16 - wie vorausgesetzt - einen Kreisbogen um die Strahlenquelle S beschreibt, sind diese Projektionen gekrümmt. In Fig 4 sind drei verschiedene Projektionen für drei verschiedene Abstände d der Trajektorie T2 von der Trajektorie T1 dargestellt:

- Wenn der Abstand d so gewählt wird, dass sich die Projektion $b2_1$ ergibt, dann ist zwar der gesamte Untersuchungsbereich zwischen T1 und T2 rekonstruierbar, jedoch ist dieser Abstand noch nicht optimal, weil er dazu führen würde, dass die Strahlendosis unnötig hoch und der rekonstruierbare Bereich unnötig verkürzt würde.

- Der optimale Abstand $d_{opt}$ - für den Fall, dass der gesamte Querschnitt des Untersuchungsbereichs 13 abgebildet werden soll - ergibt sich dann, wenn die Projektion der Trajektorie T2 durch die Ecken der Detektoreinheit verläuft - wie bei der Projektion $b2_0$. Damit kann der Bereich zwischen den Trajektorien ebenfalls noch abgebildet werden, und der Abstand d ist größer als bei der Pojektion $b2'_1$.

- Wenn der Abstand noch größer gewählt wird, ergibt sich die Projektion $b2_2$ Diese Projektion schneidet nicht mehr die seitlichen Ränder der Detektoreinheit (die in Fig 4 oben und unten liegen), sondern nur noch den rechten Rand. In diesem Fall ist es nicht mehr möglich, den gesamten Querschnitt des Untersuchungsbereichs zwischen den beiden Trajektorien vollständig zu rekonstruieren.

- Wenn man jedoch ein Objekt untersucht mit einem geringeren Durchmesser als der Untersuchungsbeceich 13 und man den Öffnungswinkel des Strahlenbündels 4 in einer zur Rotationsachse senkrechten Ebene entsprechend einengt, dann kann auch in diesem Falle noch eine vollständige Rekonstruktion der Absorptionsverteilung in dem in seinem Durchmesser reduzierten Untersuchungsbereich möglich sein. Vorraussetzung dafür ist, dass das Strahlenbündel 4 nur noch den Teil der Detektoreinheit bestrahlt, dessen Grenzen mit Ro und Ru bezeichnet sind, so dass die Projektion b2$_2$ gerade durch die Eckpunkte des für die Akquisition von Meßdaten benutzten Bereiches der Detektoreinheit verläuft.

Der optimale Abstand d$_{opt}$, für den eine vollständige Rekonstruktion der Absorptionsverteilung möglich ist, berechnet sich demgemäß nach der Gleichung

$$d_{opt} = s \, \tan\gamma \, \cos\beta$$

Dabei ist s der Abstand der Strahlenquelle S von der Rotationsachse, γ der Konuswinkel (das ist der halbe Öffnungswinkel des Strahlenbündels 4 in einer die Rotationsachse 14 enthaltenden Ebene) und β der Fächerwinkel (das ist der halbe Öffnungswinkel des Strahlenbündels 4 in einer zur Rotationsachse 14 senkrechten Ebene).

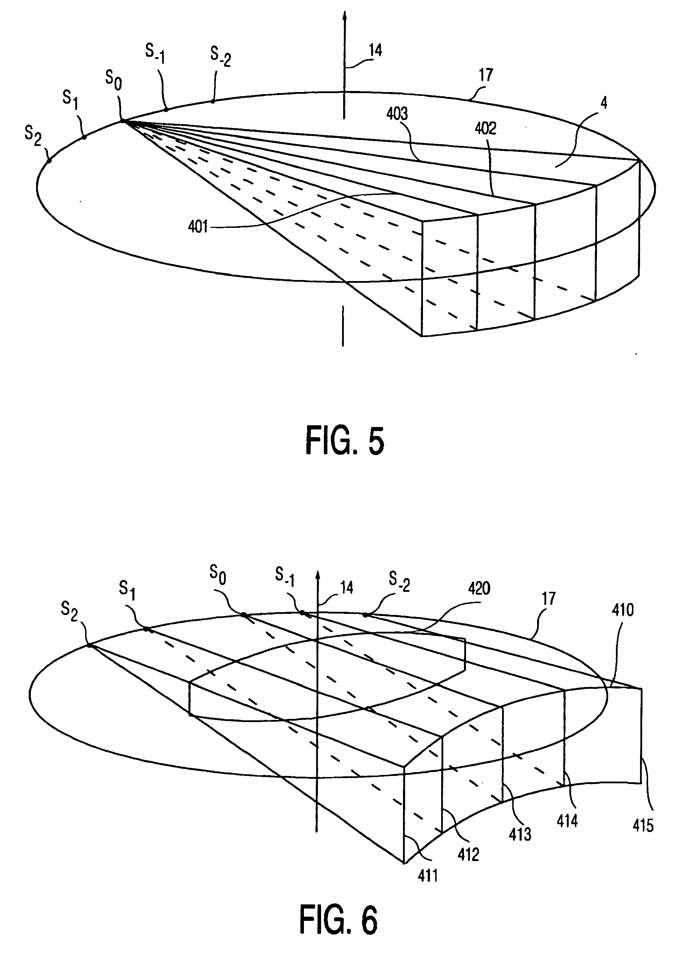

Fig. 5 zeigt die kreisförmige Bahn 17 (z.B. die Trajektorie T1), auf der sich die Strahlenquelle S und die Detektoreinheit 16 um die Rotationsachse 14 bewegen. Für eine bestimmte Strahlenquellenpostion S$_0$ ist das Strahlenbündel 4 dargestellt. Man kann sich dieses kegelförmige Strahlenbündel 4 aus einer Vielzahl von ebenen Strahlenfächern zusammengesetzt denken, die sich - wie die Strahlenfächer 401...403 - in zur Rotationsachse 14 parallelen Ebenen befinden. Obwohl man die Strahlen des kegelförmigen Strahlenbündels 4 auch auf andere Weise zusammenfassen kann, soll der Begriff "Strahlenfächer" im folgenden nur für solche Strahlen verwendet werden, die in einer gemeinsamen zur Rotationsachse parallelen Ebene liegen. Diese Strahlenfächer gehen von einer Strahlenquellenpostion aus und werden von je einer zur Rotationsachse 14 parallelen Spalte von Detektorelementen auf der Detektoreinheit 16 erfaßt. Fig. 5 deutet an, dass auch in anderen Positionen der Strahlenquelle (z.B. S$_{-1}$, S$_1$ oder S$_2$) das emittierte, kegelförmige Strahlenbündel 4 gemessen wurde.

In den Schritten 104 bis 107 erfolgt ein Rebinning Die auf beiden Trajektorien gewonnenen Daten werden dabei so umsortiert und uminterpoliert als wären sie mit einer anderen Strahlenquelle (einer kreisbogenförmigen Strahlenquelle die zueinander parallele Strahlenfächer emittiert) und mit einem anderen Detektor (einem ebenen, rechteckigen und die Rotationsachse 14 enthaltenden "virtuellen" Detektor) gemessen worden.

Im Schritt 104 werden dazu zunächst die Strahlenfächer von verschiedenen Strahlenquellenpositionen, die in zueinander parallelen Ebenen liegen, zu jeweils einer Gruppe zusammengefaßt. Die zu einer Gruppe gehörenden Strahlenfächer erfüllen daher die Bedingung, dass die Summe der Winkel χ und δ für alle Strahlenfächer dieser Gruppe den gleichen Wert hat. Dabei ist χ der Winkel, den die Ebene des Strahlenfächers mit einer durch die Strahlenquellenposition und die Rotationsachse definierten Ebene einschließt und der durch die Lage der Spalte von Detektorelementen gegeben ist, die den betreffenden Strahlenfächer erfaßt hat. δ ist ein die Strahlenquellenposition (z.B. S$_0$) auf dem Kreis 17 kennzeichnender Winkel. Wenn die Strahlenfächer für eine Strahlenquellenposition diese Bedingung nicht exakt erfüllen, muß für diese Strahlenquellenposition ein entsprechender Strahlenfächer durch Interpolation aus den Strahlen benachbarter Strahlenfächer ermittelt werden.
Fig. 6 zeigt eine auf diese Weise gebildete Gruppe von Strahlenfächern. Von jeder der einander benachbarten Strahlenquellenpositionen S$_{-2}$..S$_0$..S$_2$ gehört jeweils ein Strahlenfächer zu einer Gruppe. Jede Gruppe kann durch den Winkel und δ der Strahlenquellenposition (z.B. S$_0$) gekennzeichnet werden, deren zu der Gruppe gehörender Strahlenfächer die Rotationsachse 14 durchsetzt (in der Regel ist dies die mittlere Strahlenquellenposition - im Beispiel nach Fig. 6 also die Strahlenquellenpostion S$_0$). Es können sich dann so viele Gruppen von Strahlenfächern ergeben, wie es Strahlenpositionen gibt, aber auch mehr oder weniger.

Die auf diese Weise bestimmten Strahlenfächer - darunter die in Fig.6 dargestellten Strahlenfächer 411...415 - definieren ein Strahlenbündel 410, das eine zeltartige Form hat und sich aus Strahlenfächern zusammensetzt, die in zueinander und zur Rotationsachse parallelen Ebenen liegen. In Fig 6 ist außerdem der Schnittbereich 420 dargestellt, der sich ergibt, wenn das Strahlenbündel 410 von einer die Rotationsachse 14 enthaltenden und zu den Ebenen der Strahlenfächer 411...415 senkrechten Ebene geschnitten wird.

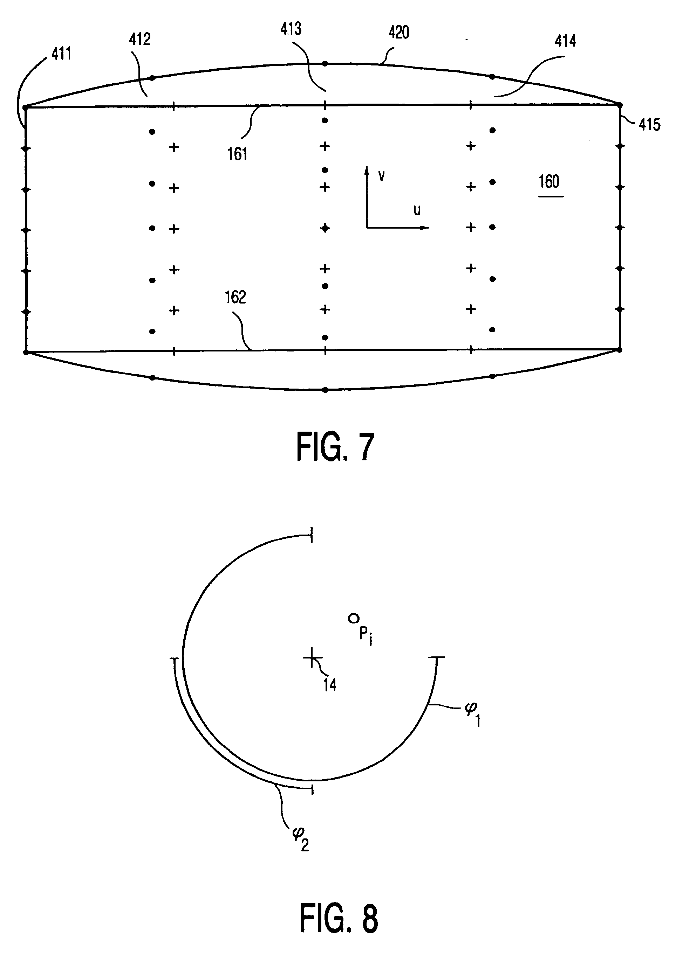

Wie insbesondere Fig. 7 zeigt, die diesen Schnittbereich darstellt, sind die oberen und unteren (in Richtung der Rotationsachse gegeneinander versetzten) Ränder gewölbt. Diese Wölbung ist darauf zurückzuführen, dass die Strahlenquellenpostionen in der Mitte (z.B. S$_0$) weiter von der Schnittebene entfernt sind, als die Strahlenquellenposition am Rande (S$_2$ oder S$_{-2}$) und dass die Fächer alle den gleichen Öffnungswinkel haben, weil die Detektorzeilen einen Kreisbogen um die Strahlenquelle S beschreiben. Bei einer anderen Grometrie der Detektorzeilen würde sich die Form der Schnittebene 420 ändern. Bei einer ebenen Detektoreinheit (z.B. mit geradlinigen Detektorzeilen) wäre die Wölbung noch ausgeprägter, weil die am Rand liegenden Strahlenfächer (wie z.B. 411 und 415) dabei einen geringeren Öffnungswinkel hätten.

Für jede Gruppe von Strahlenfächern wird in dem ebenen Schnittbereich 420 ein rechteckiger virtueller Detektor definiert (Schritt 105) dessen oberer und unterer Rand 161 bzw. 162 durch die Abmessungen der äußeren Strahlenfächer 411 bzw. 415 in den ebenen Schnittbereich gegeben sind. Wenn die Trajektorien den

optimalen Abstand $d_{opt}$

voneinander haben, berühren sich z.B der Rand 161 eines virtuellen Detektors der einen Trajektorie gerade den Rand 162 eines virtuellen Detektors der anderen Trajektorie.

Wenn der Durchmesser des zu rekonstruierenden FOV kleiner gewählt worden ist als der Durchmesser des Untersuchungsbereichs 13, können von jeder Gruppe jeweils die äußeren Strahlenfächer (z.B. 411, 415) entfallen. Der Abstand der oberen und unteren Ränder 161 und 162 - und damit der optimale Abstand $d_{opt}$ der Trajektorien T1 und T2 - kann in diesem Falle größer gewählt werden als bei dem in Fig. 7 dargestellten Beispiel.

In Fig. 7 sind außerdem - durch nmde Punkte markiert - die Durchstoßpunkte einiger in den Strahlenfächern 411...415 enthaltener Strahlen durch diesen virtuellen Detektor dargestellt. Schließlich sind durch Kreuze die Stützpunkte eines regelmäßigen kartesischen Gitters angedeutet. Die Durchstoßpunkte und die Stützpunkte fallen in der Regel nicht zusammen. Man erkennt, dass die Strahlenfächer außen enger beieinander liegen als innen (402 zB. liegt näher an 411 als an 413) und dass die Durchstoßpunkte der Strahlen eines Strahlenfächers innen (z.B. bei 413) weiter voneinander entfernt sind, als außen (z.B. 415). Aus den Meßwerten für die Durchstoßpunkte müssen daher in den beiden folgenden Schritten 106 und 107 die Meßwerte an den äquidistanten Stützstellen innerhalb des virtuellen Detektors 160 ermittelt werden.

Im Schritt 106 erfolgt dabei zunächst eine vertikale Interpolation derart, dass die Stützstellen in vertikaler Richtung alle Strahlenfächer voneinander den gleichen Abstand haben, wie die Durchstoßpunkte bzw. Stützstellen am Rand des virtuellen Detektors 160. Im Schritt 107 erfolgt eine Interpolation in horizontaler Richtung, so dass man interpolierte Werte für Stützstellen enthält, die innerhalb des virtuellen Detektors 160 in horizontaler Richtung voneinander den gleichen Abstand haben. - Die Interpolationsschritte 106 und 107 können auch in umgekehrter Reihenfolge ausgeführt und ggf. sogar miteinander kombiniert werden.

Das Ergebnis dieser Interpolation in horizontaler und vertikaler Richtung ist, dass innerhalb des virtuellen Detektors 160 die Intensität der Strahlung an den äquidistanten Stützpunkten eines regelmäßigen kartesischen Gitters in dem u,v-Koordinatonsystem des virtuellen Detektors 160 vorliegt. Die Stützstellen definieren ihrerseits wiederum neue Strahlenfächer, die in parallelen Ebenen mit jeweils dem gleichen Abstand voneinander liegen. Nur diese neuen Strahlenfächer (die teilweise mit den originalen Strahlenfächern identisch sein können) werden für die weitere Rekonstruktion benutzt.

Ein Teil der Strahlen, deren Durchstoßpunkte außerhalb des virtuellen Detektors 160 liegen, werden in den Schritten 106 und 107 nicht genutzt. Es ist daher von Vorteil, den Kollimator so zu gestalten, dass das kegelförmige Strahlenbündel 4 von vornherein keine Strahlen enthält, die oberhalb des oberen Randes 161 oder unterhalb des unteren Randes 162 des virtuellen Detektors 160 verlaufen. Dadurch wird die Strahlenbelastung für den Patienten weiter verringert.

Anstelle von geradlinigen, senkrecht zur Rotationsachse verlaufenden Kanten, müßte die Kollimatoranordnung 3 zu diesem Zweck nach innen gewölbte Kanten aufweisen, so dass die Strahlenfächer, die die Rotationsachse 14 schneiden oder davon einen geringen Abstand haben, einen geringeren Öffnungswinkel (gemessen in einer zur Rotationsachse 14 parallelen Ebene) haben, als Strahlenfächer am äußeren Rand des Strahlenbündels. Der äußere Rand, der von einem solchen Strahlenbündel auf der Detektoreinheit 14 bestrahlten Fläche würde dann mit der Projektion $b2_0$ (vgl. Fig 4) zusammenfallen, die durch den Eckpunkt des für die Akquisition von Meßdaten benutzten Bereichs der Detektoreinheit verläuft.

Anschließend werden im Schritt 108 die den einzelnen Strahlen zugeordneten Daten mit einem Wichtungsfaktor multipliziert, der dem Cosinus des Winkels entspricht, den der Strahl mit einem Lot auf den virtuellen Detektor 160 einschließt. Dadurch wird die Bildqualität verbessert.

Nach den Schritten 103 bis 108 ist somit für jede Gruppe von Strahlenfächern die Strahlenintensität an den regelmäßigen Stützstellen des zu dieser Gruppe gehörenden virtuellen Detektors 160 bestimmt. Dies edeichtert die erforderliche Hochpaßfilterung fundamental, weil ledigich eine eindimensionale Filterung der durch das Rebinning auf den virtuellen Detektor 160 erzeugten Daten notwendig ist. Im Schritt 109 wird daher auf diese Daten eine eindimensionale Filterung mit einem rampenförmig mit der Frequenz ansteigenden Übertragungsfaktor angewandt. Dazu werden jeweils lediglich die Werte von in horizontaler Richtung aufeinanderfolgenden Stützstellen herangezogen. Diese Filterung wird für alle Gruppen der durch das Rebinning entstandenen Strahlenfächer ausgeführt.

Die nach dem Rebinning und der Filterung für die durch die Stützstellen im virtuellen Fenster 160 definierten Strahlen ermittelten Daten werden anschließend zur Rekonstruktion der Absorptionsverteilung im Untersuchungsbereich durch eine Rückprojektion herangezogen.

Nach der Vorgabe eines Voxels P(x,y,z) im Schritt 110 wird im Schritt 111 ein Winkel φ vorgegeben, den die Strahlenfächer in einer zur Rotationsachse senkrechten Ebene definieren. Im Schritt 112 wird dann geprüft, ob es einen von der ersten Trajektorie T1 ausgehenden Strahl gibt, der unter dem Winkel $φ_1=φ$ genau durch das Voxel P(x,y,z) verläuft. Ist dies der Fall, dann wird im Schritt 113 für dieses Voxel P(x,y,z) ein Beitrag akkumuliert, der dem für diesen Strahl aus der Filteroperation im Block 109 hervorgehenden Wert entspricht. Es kann auch sein, dass keiner der Strahlen der durch den Winkel $φ_1$ definierten Gruppe exakt durch das Voxel P verläuft, dass aber mehrere Strahlen das Voxel umschließen. In diesem Fall wird der dem Punkt zuzuordnende Beitrag durch eine geeignete Interpolation der diesen Strahlen zugeordneten Werte ermittelt.

Nach dem Schritt 113 - bzw. wenn die Prüfung im Schritt 112 ein negatives Ergebnis hatte - wird im Schritt 114 geprüft, ob es einen von der zweiten Trajektorie T2 ausgehenden Strahl gibt, der unter dem Winkel $\varphi_2 = \varphi$ durch das Voxel P(x,y,z) verläuft bzw ob es mehrere Strahlen gibt, die unter diesem Winkel das Voxel umschließen. Ist dies der Fall, dann wird im Schritt 115 für dieses Voxel P(x,y,z) ein Beitrag akkumuliert, der dem für diesen Strahl (bzw diese Strahlen) aus der Filteroperation im Block 109 hervorgehenden Wert (bzw Werten) entspricht.

Nachdem der Beitrag zu dem Voxel P für die Richtung $\varphi$ ermittelt worden ist, werden die Schritte 112 - 115 für einen anderen Winkel $\varphi$ wiederholt. Nachdem für $\varphi$ ein Winkelbereich von 360° durchlaufen ist, werden die Schritte 111 - 115 für ein anderes Voxel P (x,y,z) wiederholt, bis für sämtliche Voxel ein Absorptionswert ermittelt worden ist.

Es sind dabei drei Fälle möglich:

- Befindet sich das Voxel innerhalb des durch die Trajektorie T1 und die Strahlenbündel $4_1$ und $4'_1$ definierten, bezüglich der Achse 14 rotationssymmetrischen, diskusähnlichen Bereich, dann ist es aus einem Winkelbereich von 360° von der Trajektorier T1 aus bestrahlt worden. Es kann dann für jeden Winkel $\varphi$ im Bereich von 0 bis 360° (im Schritt 113) ein Beitrag zum Absorptionswert des Punktes P (x,y,z) ermittelt werden (und zu den vorher für dieses Voxel ermittelten Beiträgen hinzu addiert werden), der ausschließlich von den auf der ersten Trajektorie akquirierten Meßwerten abgeleitet wurde.

- Befindet sich das Voxel innerhalb des durch die Trajektorie T2 und die Strahlenbündel $4_2$ und $4'_2$ definierten, bezüglich der Achse 14 rotationssymmetrischen, diskusähnlichen Bereich, dann ist es aus einem Winkelbereich von 360° von der Trajektorie T2 aus bestrahlt worden. Es kann dann für jeden Winkel $\varphi$ im Bereich von 0 bis 360° (im Schritt 115) ein Beitrag zum Absorptionswert des Punktes P (x,y,z) ermittelt werden, der ausschließlich von den auf der zweiten Trajektorie T2 akquirierten Meßwerten abgeleitet wurde.

- Liegt das Voxel $P_i$ jedoch außerhalb der erwähnten diskusförmigen Bereiche in dem Zwischenbereich Z - wie zB. das Voxel $P_i$ in Fig 3 - dann ist dieses Voxel nicht von sämtlichen Strahlenquellenpositionen auf der Trajektorie T1 oder der Trajektorie T2 aus bestrahlt worden. Die Beiträge zum Absorptionswert des Voxels P (x,y,z) können jedoch aus den auf beiden Trajektorien akquirierten Meßwerten abgeleitet werden.

Der letzgenannte Fall ist in Fig 8 dargestellt, die die senkrecht zur Zeichenebene verlaufende Rotationsachse 14 und das Voxel $P_i$ zeigt. Außerdem ist der Bogen $\varphi_1$ dargestellt, von dem aus die Strahlenquelle auf der Trajektorie T1 das betreffenden Voxel bestrahlen kann. Man erkennt, dass das Voxel $P_i$ aus den dichter bei diesem Punkt liegenden Strahlenquellenposition nicht bestrahlt werden kann. Auch aus Fig. 3 sieht man, dass der Punkt $P_i$ zwar aus der unteren Strahlenquellenpositionen von dem Strahlenbündel $4'_1$ erfaßt werden kann, nicht aber von dem Strahlenbündel $4_1$, das in der dichter beim Punkt $P_i$ liegenden Strahlenquellenposition $S_1$ emittiert wird.

Schließlich ist in Fig 8 noch der Winkelbereich $\varphi_2$ angegeben, von dem aus das Voxel $P_i$ auf der Trajektorie T2 bestrahlt wird (eigentlich müßte dieser Bogen den selben Durchmesser haben, wie der Bogen $\varphi_1$; der Übersichtlichkeit halber ist der Radius des Bogens aber geringfügig vergrößert dargestellt). Wenn das Voxel $P_i$ dichter bei der Trajektorie T1 liegt, ist der Bogen $\varphi_1$ länger als der Bogen $\varphi_2$. Liegt $P_i$ genau in der Mitte zwischen beiden Trajektorien, dann sind beide Bögen gleich lang und erstrecken sich über je 180°. Wenn $P_i$ dichter an der Trajektorie T2 liegt, wird $\varphi_2$ größer als $\varphi_1$.

Wenn der Abstand zwischen den Trajektorien gemäß der Gleichung 5 gewählt ist, dann ist die Summe der Bogenwinkel (von der Rotationsachse aus gesehen) genau 360°. Deshalb und weil es nicht darauf ankommt, ob der Punkt $P_i$ von links unten nach rechts oben oder von rechts oben nach links unten durchstrahlt wird, kann man die Meßdaten für das Voxel $P_i$, die auf der ersten Trajektorie erfaßt wurden, durch Daten ergänzen, die auf der zweiten Trajektorie unter dein Winkel $\varphi_2$ gemessen wurden (mit $\varphi_2 = \varphi_1°$). Für ein Voxel im Zwischenbereich Z können sich daher Beiträge sowohl aus dem Schritt 113 als auch aus dem Schritt 115 ergeben. Es ist aber auch möglich, dass nur in einem der Schritte 113, 115 ein Beitrag geliefert wird oder kein Beitrag

Nachdem auf die geschilderte Weise die Absorptionsverteilung in sämtlichen Voxeln P(x,y,z) der beiden diskusförmigen Bereiche und in dem Zwischenbereich rekonstruiert worden ist, kann das Verfahren beendet werden (Schritt 116). Es ist aber auch möglich, zusätzlich die Absorptionsverteilung in den sich außen anschließenden Bereichen zu rekonstruieren, die in Fig. 3 gestrichelt angedeutet sind Ein geeignetes Verfahren hierzu ist in der eingangs erwähnten deutschen Patentanmeldung (PHD 98-111) beschrieben. Allerdings können bei der Rekonstruktion in diesem Bereich mehr Artefakte auftreten und das Signal/Rausch-Verhältnis ist ungünstiger als in den diskusförmigen Bereichen und im Zwischenbereich.

Wenn der für die Untersuchung relevante Bereich größer ist, können die Meßdaten entlang von mehr als zwei Trajektorien rekonstruiert werden. Der Bereich zwischen zwei benachbarten Trajektorien wird dann jeweils so rekonstruiert, wie in Verbindung mit Fig. 2 erläutert.

Es sind auch andere Rekonstruktionserfahren möglich, bei denen das Rebinning auf andere Weise erfolgt. Auch hier müssen jedoch zur Rekonstruktion der Absorptionsverteilung in dem Zwischenbereich Meßdaten herangezogen werden, die entlang beider Trajektorien akquiriert wurden.

**Patentansprüche**

1. Computertomographie-Verfahren mit den Schritten

- Erzeugen eines kegelförmigen, einen Untersuchungsbereich (13) bzw. ein darin befindliches Objekt durchsetzenden Strahlenbündels (4) mit einer Strahlenquelle (S),
- Erzeugung von Relativbewegungen zwischen der Strahlenquelle (S) einerseits und dem Untersuchungsbereich (13) bzw. dem Objekt andererseits, die eine Rotation um eine Rotationsachse (14) entlang einer ersten, geschlossenen Trajektorie (T1) und entlang wenigstens einer zweiten mit der ersten identischen, jedoch in Richtung der Rotationsachse versetzten Trajektorie (T2) umfassen
- Akquisition von Meßdaten, die von der Intensität in dem Strahlenbündel(4) jenseits des Untersuchungsbereiches (13) abhängen, mit einer Detektoreinheit (16) während der Relativbewegungen,
- Rekonstruktion der Absorptionsverteilung im Untersuchungsbereich (13),

**gekennzeichnet durch**

- eine solche Wahl des Abstandes (d) der Trajektorien (T1, T2) voneinander, dass
- einerseits in einem Zwischenbereich (Z) zwischen den Trajektorien Voxel ($P_i$) vorhanden sind, die bei beiden Relativbewegungen zeitweise nicht von Strahlung getroffen werden, und
- andrerseits die Strahlenquelle (S) beide Trajektorien (T1, T2) stets derart auf die Detektoreinheit projiziert, dass die Projektionen ($b2_0$) den seitlichen Rand des für die Akquisition von Meßdaten benutzten Bereiches der Detektoreinheit (16) schneiden,
- und **durch** eine Rekonstruktion der Absorptionsverteilung im Zwischenbereich (Z), bei der Meßdaten herangezogen werden, die während der beiden Relativbewegungen akquiriert wurden.

2. Computertomographie-Verfahren nach Anspruch 1, **gekennzeichnet durch** eine solche Wahl des Abstandes (d) der Trajektorien voneinander, dass die Projektion ($b2_0$, $b2_2$) einer der beiden Trajektorien stets **durch** wenigstens einen Eckpunkt des für die Akquisition von Meßdaten benutzten Bereiches der Detektoreinheit verlaufen.

3. Computertomographie-Verfahren nach Anspruch 1, **dadurch gekennzeichnet,** **dass** die Rekonstruktion folgende Schritte umfaßt:

a) Rebinning der Meßdaten zu einer Anzahl von Gruppen, wobei jede Gruppe mehrere zueinander und zur Rotationsachse parallele Ebenen umfaßt, in denen sich je ein Strahlenfächer (411...415) befindet,
b) eindimensionale Filterung der durch das Rebinning erzeugten Daten einer jeden Gruppe in Richtung senkrecht zur Richtung der Ebenen,
c) Rekonstruktion der räumlichen Verteilung der Absorption durch Rückprojektion der gefilterten Daten von mehreren Gruppen unter Heranziehung gefilterter Daten von beiden Trajektorien für die Rückprojektion im Zvdschenbereich (Z).

4. Computertomographie-Verfahren nach Anspruch 3, **dadurch gekennzeichnet,** **dass** das Rebinning auf je einen zu den Ebenen einer jeden Gruppe senkrechten, ebenen und rechteckigen virtuellen Detektor (160) erfolgt, der die Rotationsachse (14) enthält.

5. Computertomograph zur Durchführung des Verfahrens nach Anspruchs 1mit

- einer Strahlenquelle,
- einer damit gekoppelten Detektoreinheit,
- einer Antriebsanordnung, um ein im Untersuchungsbereich enthaltenes Objekt und die Strahlenquelle relativ zueinander um eine Rotationsachse rotieren und/oder sich parallel zur Rotationsachse verschieben zu lassen,
- einer Rekonstruktionseinheit zur Rekonstruktion der räumlichen Verteilung der Absorption innerhalb des Untersuchungsbereiches aus den von der Detektoreinheit akquirierten Meßdaten, und
- einer Steuereinheit zur Steuerung der Strahlenquelle, der Detektoreinheit, der Antriebsanordnung und der Rekonstruktionseinheit
- Mittel (S, 3) zum Erzeugen eines kegelförmigen, einen Untersuchungsbereich bzw. ein darin befindliches Objekt durchsetzenden Strahlenbündels mit einer Strahlenquelle(S),
- eine erste Antriebseinheit (2) zur Erzeugung von eine Rotation um eine Rotationsachse (14) enthaltenden Relativbewegungen zwischen der Strahlenquelle einerseits und dem Untersuchungsbereich (13) bzw. dem Objekt andrerseits entlang einer ersten, geschlossenen Trajektorie (T1) und entlang wenigstens einer zweiten mit der ersten identischen, jedoch in Richtung der Rotationsachse versetzten Trajektorie (T2),
- eine zweite Antriebseinheit (5) zur Verschiebung des Objektes relativ zur Strahlenquelle (S) zwischen den Relativbewegungen in Richtung parallel zur Rotationsachse (14) um eine Strecke (d),

**dadurch gekennzeichnet,**
**dass** einerseits in einem Zwischenbereich (Z) zwischen den Trajektorien (T1, T2) Voxel (P$_i$) vorhanden sind, die bei beiden Relativbewegungen zeitweise nicht von Strahlung getroffen werden, und andererseits die Strahlenquelle (S) beide Trajektorien stets derart auf die Detektoreinheit projiziert, dass die Projektionen den seitlichen Rand des für die Akquisition von Meßdaten benutzten Bereiches der Detektoreinheit (16) schneiden,

- und durch Mittel (10) zur Rekonstruktion der Absorptionsverteilung im Untersuchungsbereich, wobei für die Rekonstruktion im Zwischenbereich Meßdaten herangezogen werden, die während der beiden Relativbewegungen akquiriert wurden.

6. Computertomograph zur Durchführung des Verfahrens nach Anspruchs 5 mit einer Kollimatoranordnung (3) zur Erzeugung des Strahlenbündels,
**dadurch gekennzeichnet,**
**dass** die in Richtung der Rotationsachse (14) gegeneinander versetzten Ränder der Kollimatoranordnung (3) so geformt sind, dass das kegelförmige Strahlenbündel in seiner Mitte eine geringere Öffnung hat als an seinen Rändern.

7. Computerprogramm für eine Steuereinheit (7) zur Steuerung einer Strahlenquelle (S), einer Detektoreinheit (16), einer Antriebsanordnung und einer Rekonstruktionseinheit (10) eines Computertomographen zur Durchführung des Verfahrens nach Anspruch 1 gemäß folgendem Ablauf:

- Erzeugen eines kegelförmigen, einen Untersuchungsbereich (13) bzw. ein darin befindliches Objekt durchsetzenden Strahlenbündels (4) mit einer Strahlenquelle (S),
- Erzeugung von eine Rotation um eine Rotationsachse enthaltenden Relativbewegungen zwischen der Strahlenquelle einerseits und dem Untersuchungsbereich bzw. dem Objekt andererseits entlang einer ersten, geschlossenen Trajektorie (T1) und entlang wenigstens einer zweiten mit der ersten identischen, jedoch in Richtung der Rotationachse versetzten Trajektorie (T2), wobei
- einerseits in einem Zwischenbereich zwischen den Trajektorien Voxel (P$_i$) vorhanden sind, die bei beiden Relativbewegungen zeitweise nicht von Strahlung getroffen wenden, und
- andererseits die Strahlenquelle beide Trajektorien stets derart auf die Detektoreinheit projiziert, dass sie den seitlichen Rand des für die Akquisition von Meßdaten benutzten Bereiches der Detektoreinheit schneiden,
- Akquisition von Meßdaten, die von der Intensität in dem Strahlenbündel jenseits des Untersuchungsbereiches abhängen, mit einer Detektoreinheit (16) während der Relativbewegungen, Rekonstruktion der Absorptionsverteilung im Untersuchungsbereich, wobei für die Rekonstruktion im Zwischenbereich Meßdaten herangezogen werden, die während der beiden Relativbewegungen akquiriert wurden.

## Revendications

1. Procédé de tomographie informatisée avec les étapes de

- production d'un faisceau de rayons (4) conique traversant une zone d'examen (13) ou un objet qui s'y trouve avec une source de rayons (S);
- production de mouvements relatifs entre la source de rayons (S), d'une part, et la zone d'examen (13) ou l'objet, d'autre part, qui comprennent une rotation autour d'un axe de rotation (14) le long d'une première trajectoire (T1) fermée et le long d'au moins une deuxième trajectoire (T2) identique mais décalée dans la direction de l'axe de rotation;
- acquisition de données de mesure qui dépendent de l'intensité dans le faisceau de rayons (4) au-delà de la zone d'examen (13), avec une unité de détecteurs (16) pendant les mouvements relatifs,
- reconstruction de la distribution de l'absorption dans la zone d'examen (13),

**caractérisé par**

- un choix tel de la distance (d) entre les trajectoires (T1, T2) que
- d'une part, il est présent dans une zone intermédiaire (Z) entre les trajectoires, des voxels (P$_i$) qui ne sont temporairement pas touchés par le rayonnement pour les deux mouvements relatifs et,
- d'autre part, la source de rayons (S) projette toujours les deux trajectoires (T1, T2) sur l'unité de détecteurs de telle sorte que les projections (b2$_0$) coupent le bord latéral de la plage de l'unité de détecteurs (16) utilisée pour l'acquisition de données de mesure,
- et par une reconstruction de la distribution de l'absorption dans la zone intermédiaire (Z) dans laquelle sont utilisées les données de mesure qui ont été acquises pendant les deux mouvements relatifs.

2. Procédé de tomographie informatisée selon la revendication 1,
   **caractérisé par**
   un choix de la distance (d) réciproque entre les trajectoires tel que la projection (b2$_0$, b2$_2$) d'une des deux trajectoires s'étende toujours par au moins un coin de la zone de l'unité de détecteurs utilisée pour l'acquisition de données de mesure.

3. Procédé de tomographie informatisée selon la revendication 1,
   **caractérisé en ce**
   **que** la reconstruction comprend les étapes suivantes :

   a) rebinning des donnée de mesure en un nombre de groupes, chaque groupe comprenant plusieurs plans parallèles l'un à l'autre et à l'axe de rotation dans lesquels se trouve à chaque fois un éventail de rayons (411 ...415),
   b) filtrage unidimensionnel des données produites par le rebinning de chaque groupe dans une direction perpendiculaire à la direction des plans;
   c) reconstruction de la distribution spatiale de l'absorption par rétroprojection des données filtrées de plusieurs groupes en utilisant des données filtrées de deux trajectoires pour la rétroprojection dans la zone intermédiaire (Z).

4. Procédé de tomographie informatisée selon la revendication 3,
   **caractérisé en ce**
   **que** le rebinning est effectué sur un détecteur virtuel plat et rectangulaire, perpendiculaire aux plans de chaque groupe qui contient l'axe de rotation (14).

5. Tomographe informatisé pour la mise en oeuvre du procédé selon la revendication 1 avec :

   - une source de rayons,
   - une unité de détecteurs couplée à celle-ci,
   - un dispositif d'entraînement pour faire tourner réciproquement un objet contenu dans la zone d'examen et la source de rayons autour d'un axe de rotation et/ou se déplacer parallèlement à l'axe de rotation,

     - une unité de reconstruction pour la reconstruction de la distribution spatiale de l'absorption dans la zone d'examen à partir des données de mesure acquises par l'unité de détecteurs et
     - une unité de commande pour la commande de la source de rayons, de l'unité de détecteurs, du dispositif d'entraînement et de l'unité de reconstruction.
   - des moyens (S, 3) pour la production d'un faisceau de rayons conique traversant une zone d'examen ou un objet qui s'y trouve avec une source de rayons (S),
   - une première unité d'entraînement (2) pour la production de mouvements relatifs contenant une rotation autour de l'axe de rotation (14) entre la source de rayons, d'une part, et la zone d'examen (13) ou l'objet, d'autre part, le long d'une première trajectoire (T1) fermée et le long d'au moins une deuxième trajectoire (T2) identique à la première mais décalée dans la direction de l'axe de rotation,
   - une deuxième unité d'entraînement (5) pour le déplacement de l'objet par rapport à la source de rayons (S) entre les mouvements relatifs dans une direction parallèle à l'axe de rotation (14) autour d'une distance (d), **caractérisé en ce que**

   d'une part, dans une zone intermédiaire (Z) entre les trajectoires (T1, T2), des voxels (P$_i$) sont présents et ne sont temporairement pas touchés par le rayonnement dans les deux mouvements relatifs et
   d'autre part, la source de rayons (S) des deux trajectoires projette toujours sur l'unité de détecteurs de telle sorte que les projections coupent le bord latéral de la zone de l'unité de détecteurs (16) utilisée pour l'acquisition des données de mesure
   et par des moyens (10) pour la reconstruction de la distribution de l'absorption dans la zone d'examen, les données de mesures qui ont été acquises pendant les deux mouvements relatifs étant utilisées pour la reconstruction dans la zone intermédiaire.

6. Tomographe informatisé pour l'exécution du procédé selon la revendication 5 avec un dispositif collimateur (3) pour la production du faisceau de rayons,
   **caractérisé en ce**
   **que** les bords du dispositif collimateur (3) décalés l'un par rapport à l'autre dans la direction de l'axe de rotation (14) sont formés de telle sorte que le faisceau de rayons conique ait dans son centre une ouverture inférieure à ses bords.

7. Programme informatisé pour une unité de commande (7) en vue de la commande d'une source de rayons (S), d'une unité de détecteurs (16), d'un dispositif d'entraînement et d'une unité de reconstruction (10) d'un tomographe informatisé en vue de l'exécution du procédé selon la revendication 1 selon la procédure suivante :

   - production d'un faisceau de rayons (4) conique traversant une zone d'examen (13) ou un objet qui s'y trouve avec une source de rayons (S);

- production de mouvements relatifs entre la source de rayons (S), d'une part, et la zone d'examen (13) ou l'objet, d'autre part, qui comprennent une rotation autour d'un axe de rotation (14) le long d'une première trajectoire (T1) fermée et le long d'au moins une deuxième trajectoire (T2) identique mais décalées dans la direction de l'axe de rotation, durant laquelle

- d'une part, dans une zone intermédiaire (Z) entre les trajectoires (T1, T2), des voxels (P$_i$) sont présents et ne sont temporairement pas touchés par le rayonnement dans les deux mouvements relatifs et
- d'autre part, la source de rayons (S) des deux trajectoires projette toujours sur l'unité de détecteurs de telle sorte que les projections coupent le bord latéral de la zone de l'unité de détecteurs (16) utilisée pour l'acquisition des données de mesure;

- acquisition de données de mesure qui dépendent de l'intensité dans le faisceau de rayons au-delà de la zone d'examen, avec une unité de détecteurs (16) pendant les mouvements relatifs,

- reconstruction de la distribution de l'absorption dans la zone d'examen (13), durant laquelle les données de mesure qui ont été acquises pendant les deux mouvements relatifs sont utilisées pour la reconstruction dans la zone intermédiaire.

**Claims**

1. A computer tomography method which includes the steps of

- generating, by using a radiation source (S), a conical radiation beam (4) that traverses an examination zone (13) or an object present therein,
- generating relative movements between the radiation source (S) on the one side and the examination zone (13) or the object on the other side, which relative movements include a rotation about an axis of rotation (14) along a first, closed trajectory (T1) and along at least a second trajectory (T2) which is identical to the first trajectory but offset in the direction of the axis of rotation,
- acquiring, by using a detector unit (16), measuring data that are dependent on the intensity in the radiation beam (4) to the other side of the examination zone (13) during the relative movements,
- reconstructing the absorption distribution in the examination zone (13),

**characterized by**

- such a choice of the distance (d) between the trajectories (T1, T2) that

- on the one hand in an intermediate region (Z) between the trajectories voxels (P$_i$) are present which are not exposed at times to any radiation during the two relative movements, and
- on the other hand the radiation source (S) projects both trajectories (T1, T2) continuously on the detector unit in such a manner that the projections (b2$_0$) intersect the lateral edge of the area of the detector unit (16) that is used for the acquisition of measuring data, and

- the absorption distribution in the intermediate region (Z) is reconstructed while taking into account measuring data acquired during the two relative movements.

2. A computer tomography method as claimed in claim 1,
**characterized by** the fact that the distance (d) between the trajectories is chosen in such a manner that the projection (b2$_0$, b2$_2$) of one of the two trajectories always extends through at least one corner point of the area of the detector unit that is used for the acquisition of measuring data.

3. A computer tomography method as claimed in claim 1,
**characterized in that** the reconstruction includes the following steps:

a) rebinning the measuring data so as to form a number of groups, each group including a plurality of planes that extend parallel to one another and to the axis of rotation and contain a respective fan beam (411 ... 415),
b) one-dimensional filtering of the data produced by the rebinning operation for each group in the direction perpendicular to the direction of the planes,
c) reconstructing the spatial distribution of the absorption by backprojection of the filtered data of a plurality of groups while taking into account filtered data from both trajectories for the backprojection in the intermediate region (Z).

4. A computer tomography method as claimed in claim 3,
**characterized in that** the rebinning operation is performed on a respective virtual detector (160) which extends perpendicularly to the planes of each group, has a flat and rectangular shape and contains the axis of rotation (14).

5. A computer tomograph for carrying out the method claimed in claim 1, including

   - a radiation source,
   - a detector unit which is coupled thereto,
   - a drive arrangement for rotating and/or displacing an object present in the examination zone and the radiation source relative to one another about an axis of rotation or parallel to the axis of rotation,
   - a reconstruction unit for reconstructing the spatial distribution of the absorption within the examination zone from the measuring data acquired by the detector unit, and
   - a control unit for controlling the radiation source, the detector unit, the drive arrangement and the reconstruction unit,
   - means (S, 3) for generating, using a radiation source (S), a conical radiation beam that traverses an examination zone or an object present therein,
   - a first drive unit (2) for generating relative movements, including a rotation about an axis of rotation (14), between the radiation source on the one side and the examination zone (13) or the object on the other side along a first, closed trajectory (T1) and along at least one second trajectory (T2) which is identical to the first trajectory but offset in the direction of the axis of rotation,
   - a second drive unit (5) for displacing the object relative to the radiation source (S) between the relative movements in the direction parallel to the axis of rotation (14) over a distance (d), **characterized in that**

      - on the one hand voxels ($P_i$) that are temporarily not exposed to any radiation during the two relative movements are present in an intermediate region (Z) between the trajectories (T1, T2), and
      - on the other hand the radiation source (S) projects both trajectories continuously on the detector unit in such a manner that the projections intersect the lateral edge of the area of the detector unit (16) that is used for the acquisition of measuring data, and **characterized by**

   - means (10) for reconstructing the absorption distribution in the examination zone, the reconstruction in the intermediate region utilizing measuring data acquired during the two relative movements.

6. A computer tomograph for carrying out the method as claimed in claim 5, including a collimator arrangement (3) for generating the radiation beam, **characterized in that** the edges of the collimator arrangement (3) that are offset in the direction of the axis of rotation (14) are shaped in such a manner that the aperture of the conical radiation beam is smaller at its center than at its edges.

7. A computer program for a control unit (7) for controlling a radiation source (S), a detector unit (16), a drive arrangement and a reconstruction unit (10) of a computer tomograph for carrying out the method claimed in claim 1 as follows:

   - generating, using a radiation source (S), a conical radiation beam (4) that traverses an examination zone (13) or an object present therein,
   - generating relative movements, including a rotation about an axis of rotation, between the radiation source on the one side and the examination zone or the object on the other side along a first, closed trajectory (T1) and along at least a second trajectory (T2) that is identical to the first trajectory but offset in the direction of the axis of rotation, where

      - on the one hand voxels ($P_i$) which are temporarily not exposed to any radiation during the two relative movements are present in an intermediate region between the trajectories, and
      - on the other hand the radiation source projects both trajectories continuously on the detector unit in such a manner that they intersect the lateral edge of the area of the detector unit that is used for the acquisition of measuring data, and

   - acquiring measuring data, using a detector unit (16), that is dependent on the intensity in the radiation beam to the other side of the examination zone during the relative movements,
   - reconstructing the absorption distribution in the examination zone, the reconstruction in the intermediate region utilizing measuring data acquired during the two relative movements.

FIG. 1

FIG. 2

FIG. 3

FIG. 4

15

**FIG. 5**

**FIG. 6**

FIG. 7

FIG. 8